# EUROPÄISCHE PATENTSCHRIFT

(11) **EP 3 757 571 B1**
(45) Veröffentlichungstag und Bekanntmachung des Hinweises auf die Patenterteilung: **20.09.2023**
(21) Anmeldenummer: 20182136.0
(22) Anmeldetag: 25.06.2020
(51) Int. Cl.: G01N 33/558

(54) **FLIESSTEST-EINHEIT, SET UND VERWENDUNG EINER FLIESSTEST-EINHEIT ZUR DURCHFÜHRUNG EINER NACHWEISREAKTION**
FLOW TEST UNIT, SET AND USE OF A FLOW TEST UNIT FOR CARRYING OUT A DETECTION REACTION
UNITÉ D'ESSAI D'ÉCOULEMENT, ENSEMBLE ET UTILISATION D'UNE UNITÉ D'ESSAI D'ÉCOULEMENT POUR METTRE EN UVRE UNE RÉACTION DE DÉTECTION

(30) Priorität: 27.06.2019 DE 102019117413
(43) Veröffentlichungstag der Anmeldung: 30.12.2020
(73) Patentinhaber: Testo SE & Co. KGaA, 79853 Lenzkirch (DE)
(72) Erfinder: Strnad, Martin, 79263 Simonswald (DE); Schnur, Andreas, 78628 Rottweil (DE); Riemer, Joel, 79874 Breitnau (DE); Wiech, Oliver, 78166 Donaueschingen (DE)
(74) Vertreter: Mertzlufft-Paufler, Cornelius

(56) Entgegenhaltungen:
- EP-A1- 2 676 606
- WO-A1-2017/089801
- DE-A1-102007 014 729
- DE-U1-202016 100 261
- US-A1- 2019 049 442

## Beschreibung

Die Erfindung betrifft eine Fließtest-Einheit, ein Set aus einer Fließtest-Einheit und einem Probenbehälter, sowie die Verwendung einer Fließtest-Einheit zur Durchführung einer Nachweisreaktion. Man kennt bereits seitliche Flusstests, die auch als laterale Fließtests bezeichnet werden (engl. lateral flow tests). Dabei handelt es sich um eine biochemische Methode zum qualitativen Nachweis von Stoffen mit für diese Stoffe spezifischen Antikörpern.

Lateral flow tests werden gerne als Nachweismethode eingesetzt, da der hierfür erforderliche Geräteaufwand sehr gering ist. Man kennt bereits lateral flow tests, die mittels eines Teststreifens durchführbar sind. Der Aufbau der besagten Teststreifen ist aus dem Stand der Technik daher auch bereits bekannt.

Die DE 20 2016 100 261 U1 beschreibet eine Vorrichtung zum visuellen Nachweis von Analyten in einer flüssigen Probe.

Die DE 10 2007 014 729 A1 beschreibt eine Vorrichtung zur Abarbeitung von Lateral Flow Immunoassay Testen.

Die WO 2017/089801 A1 behandelt einen diagnostischen Apparat für einen Lateral Flow Test.

Die EP 2 676 606 A1 beschreibt ein Testgerät zum Testen einer menschlichen oder tierischen Probe wie beispielsweise Blut oder Blutkomponenten.

Die US 2019/0049442 A1 beschreibt ein Testgerät zum Testen von flüssigen Proben.

Der Einsatz der Teststreifen ist zwar grundsätzlich wenig aufwändig. Soll jedoch mit Hilfe der Teststreifen infektiöses und/oder gesundheitsgefährdendes Material auf das Vorhandensein eines bestimmten Stoffes, insbesondere eines Proteins, untersucht werden, so muss die mittels der Teststreifen durchgeführte Nachweisreaktion in einem Labor mit einer bestimmten Schutzstufe durch speziell geschultes Personal erfolgen. Andernfalls ist zu befürchten, dass es zu einer Kontamination der Umgebung und einer Gefährdung von in der Umgebung befindlichen Personen kommt.

Da es bei manchen Nachweisen häufig wünschenswert ist, möglichst unmittelbar ein Ergebnis einer Nachweisreaktion zu erfahren, sind vorbekannte Teststreifen nicht dazu geeignet, um am Ort einer Probenentnahme direkt eine Nachweisreaktion durchführen zu können. Die Probe muss hier erst entnommen und in ein zur Durchführung der Nachweisreaktion geeignetes Labor befördert werden. Somit besteht ein Nachteil darin, dass es häufig sehr lange dauert, bis man ein Ergebnis der Nachweisreaktion erhält.

Es wäre daher besonders wünschenswert, wenn es die Möglichkeit gäbe, eine solche Nachweisreaktion in einem abgeschlossenen System, vorzugsweise direkt am Ort der Probenentnahme, durchführen zu können, ohne dass dabei die Gefahr einer Kontamination entsteht. Besonders wünschenswert wäre es daher, wenn die Nachweisreaktion in einem abgeschlossenen System, insbesondere hermetisch abgeschlossenen System, durchführbar ist, aus welchem kein potentiell gesundheitsgefährdendes Probenmaterial nach außen gelangen kann.

Es besteht somit die Aufgabe, eine verbesserte Möglichkeit zur Durchführung einer Nachweisreaktion zu schaffen.

Die Lösung dieser Aufgabe wird erfindungsgemäß durch die Merkmale nach Anspruch 1 bereitgestellt.

Insbesondere wird zur Lösung der Aufgabe eine Fließtest-Einheit vorgeschlagen, mit einem nach außen geschlossenen Gehäuse, in welchem sich wenigstens ein Teststreifen befindet, insbesondere eingehaust ist, wobei das Gehäuse wenigstens eine Zuführöffnung oder genau eine Zuführöffnung aufweist, und wobei der Teststreifen innerhalb des Gehäuses so angeordnet ist, dass ein Benetzungsbereich mit über die wenigstens eine Zuführöffnung eindringender Flüssigkeit benetzbar ist. Somit ist es möglich, eine Nachweisreaktion mithilfe der Fließtest-Einheit in einem abgeschlossenen System vornehmen zu können, so dass das Risiko einer Kontamination der Umgebung minimiert ist. Durch das Gehäuse ist vorzugsweise eine flüssigkeitsdichte oder hermetisch abgedichtete Kapselung des Teststreifens möglich. Wenigstens kann durch das Gehäuse in Kopplungsstellung mit einem Probenbehälter ein nach außen flüssigkeitsdichtes abgeschlossenes System hergestellt sein.

Der Teststreifen selbst ist bereits aus dem Stand der Technik bekannt. Man kennt bereits Teststreifen, die aus wenigstens einer Fließlage auf einem Träger ausgebildet sind. Die Fließlage kann beispielsweise einen Reaktionspartner und/oder Wechselwirkungspartner einer nachzuweisenden Substanz, wie z.B. einen Antikörper, aufweisen, über welchen eine Nachweisreaktion durchführbar ist.

Nachfolgend werden vorteilhafte Ausgestaltungen der Erfindung beschrieben, die allein oder in Kombination mit den Merkmalen anderer Ausgestaltungen optional zusammen mit den Merkmalen nach Anspruch 1 kombiniert werden können.

Um die Fließtest-Einheit mit einem eine zu analysierende Probe enthaltenden Behältnis verbinden zu können, weist das Gehäuse an einer Außenseite eine Kupplungsstelle auf, über welche die Fließtest-Einheit mit einer dazu passenden Gegenkupplungsstelle einer Wandung eines Probenbehälters koppelbar ist. Somit kann eine feste, insbesondere irreversible Kopplung mit weiteren Behältnissen eingerichtet werden.

Gemäß einer vorteilhaften Ausgestaltung kann der wenigstens eine Teststreifen in einen Benetzungsbereich und einen Analysebereich unterteilt sein. Dabei kann eine Nachweisreaktion in Gang setzbar sein, wenn der Benetzungsbereich mit einer zu analysierenden Flüssigkeit in Kontakt tritt. Beispielsweise kann der Teststreifen zur Durchführung eines lateralen Fließtests (Lateral Flow Test) ausgestaltet sein. So ist es möglich, relativ schnell eine Analyse einer bestimmten Probe auf das Vorhandensein eines bestimmten Stoffes durchzuführen. Besonders vorteilhaften kann dabei sein, dass die besagte Analyse am Ort der Probenentnahme mithilfe der Fließtest-Einheit durchführbar ist. Darüber hinaus ist es möglich, dass die Probe jederzeit in einem von der Umgebung abgeschlossenen Raum verbleibt, wobei der Raum mit dem Gehäuseinneren der Fließtest-Einheit verbindbar ist, so dass ein gegenüber der Umgebung geschlossenes System vorliegt. Erfindungsgemäß weist das Gehäuse ein Druckausgleichsmittel auf. Das Druckausgleichsmittel ist erfindungsgemäß als ein Auffangsack ausgestaltet. Ergänzend oder alternativ dazu ist das Druckausgleichsmittel dadurch ausgebildet, dass das Gehäuse mit einem Unterdruck versehen ist. Dies hat den Vorteil, dass ein Einströmen von Flüssigkeit in das Gehäuse befördert werden kann, insbesondere sobald eine Kopplung der Fließtest-Einheit mit einem Probenbehälter erfolgt.

Um eine möglichst einfache und kostengünstige Fertigung der Fließtest-Einheit erreichen zu können, kann es gemäß einer Weiterbildung vorgesehen sein, dass das Gehäuse zumindest zweiteilig oder genau zweiteilig ausgebildet ist. Dies erlaubt eine industrielle Massenfertigung. Beispielsweise kann das Gehäuse aus wenigstens zwei insbesondere als Schalen ausgebildete Gehäuseteilen zusammengesetzt sein. Der Teststreifen kann dabei zwischen den wenigstens zwei Gehäuseteilen eingelegt sein.

Gemäß einer weiteren vorteilhaften Ausgestaltung kann vorgesehen sein, dass der wenigstens eine Teststreifen durch wenigstens einen Haltesteg des Gehäuses gehalten ist und/oder seitlich fixiert ist. Vorzugsweise kann die Halterung und/oder die Fixierung derart eingerichtet sein, dass der wenigstens eine Teststreifen am wenigstens einen Haltesteg anliegt. Alternativ oder ergänzend dazu kann der Teststreifen derart durch einen Haltesteg gehalten werden, dass er dabei nicht durch den wenigstens einen Haltesteg deformiert ist. Somit kann ein ungehindertes Durchströmen einer Flüssigkeit durch den Teststreifen sichergestellt werden, um das Testergebnis nicht zu verfälschen. Am Gehäuse können mehrere Haltestege ausgebildet sein. Der Teststreifen kann dabei zwischen einem Paar oder mehreren Paaren von Haltestegen und/oder zwischen einer Innenwandung des Gehäuses und jeweils wenigstens einem Haltesteg angeordnet sein.

Um verhindern zu können, dass es nach einer Kopplung der Fließtest-Einheit beispielsweise mit einem Probenbehälter zu einem schwallartigen Einströmen kommt, insbesondere ausgelöst durch einen Druckausgleich zwischen einem Probenaufnahmeraum des Probebehälters und dem Gehäuseinneren der Fließtest-Einheit, kann das Gehäuse im Inneren einen Prallschutz aufweisen, durch welchen das Gehäuse in zwei Kammern unterteilt ist. Wird zwischen der Fließtest-Einheit und einem Probenbehälter eine Flüssigkeitsverbindung durch Kopplung der beiden aufgebaut, so strömt Flüssigkeit aus dem Probenbehälter in die Fließtest-Einheit. Dabei kann es vorkommen, dass die Flüssigkeit Bereiche des Teststreifens benetzt, die an sich bei ordnungsgemäßer Durchführung einer Nachweisreaktion mit dem Teststreifen nicht dazu vorgesehen sind, um direkt in die Flüssigkeit eingetaucht zu werden. Somit kann es durch das stoßartige Einströmen der Flüssigkeit und die damit verbundene fehlerhafte Benetzung des Teststreifens zu einer Verfälschung des Messergebnisses kommen. So kann es besonders zweckmäßig sein, wenn der Benetzungsbereich des wenigstens einen Teststreifens in einer Einströmkammer des Gehäuses angeordnet ist und der Analysebereich des wenigstens einen Teststreifens in einer Analysekammer des Gehäuses angeordnet ist. Somit kann gewährleistet werden, dass lediglich der Benetzungsbereich des Teststreifens mit Flüssigkeit in Verbindung kommt, da sich nach Einstellung des Druckausgleichs der Flüssigkeitspegel innerhalb des Gehäuses unterhalb des Prallschutzes befindet. Der Prallschutz kann beispielsweise durch die hierin beschriebenen Haltestege des Gehäuses ausgebildet sein.

Um ein Ergebnis der Analyse ablesen zu können, ohne dass das Gehäuse der Fließtest-Einheit geöffnet werden muss, kann das Gehäuse wenigstens teilweise transparent ausgebildet sein. Beispielsweise kann das Gehäuse wenigstens ein transparentes Sichtfenster aufweisen, über welches ein Ergebnis, beispielsweise eine Farbreaktion, ablesbar ist. Alternativ oder ergänzend dazu kann das Gehäuse, insbesondere an der Außenseite, eine Markierung aufweisen, über welche durch einen Anwender ein Ergebnis einer Analyse ablesbar ist. Bei der Markierung kann es sich beispielsweise um eine Skala und/oder wenigstens einen Farbreferenzwert handeln. Somit ist eine besonders einfache Auswertung einer mittels der Fließtest-Einheit vorgenommen Analyse möglich, insbesondere ohne dass weiteres Equipment und/oder Referenzen, z.B. zum Abgleich mit einer Farbereaktion zur Bestimmung des Ergebnisses, erforderlich sind.

Um eine Flüssigkeitsverbindung mit einem weiten Behältnis, beispielsweise einem Probenbehälter, herstellen zu können, kann die wenigstens eine Zuführöffnung als ein Durchbruchelement ausgebildet sein. Insbesondere kann die Zuführöffnung als eine radial und/oder axial abragende Nase ausgebildet sein. Mittels des Durchbruchelements kann eine Sollbruchstelle in einer Wandung, beispielsweise eines Probenbehälters, durch einen Kopplungsvorgang der Fließtest-Einheit mit der Wandung durchbrochen werden. Dies kann derart erfolgen, dass über die Zuführöffnung eine Flüssigkeitsverbindung mit dem Probenbehälter hergestellt ist.

Gemäß einer weiteren vorteilhaften Ausgestaltung kann ein Druckausgleichsmittel, beispielsweise das bereits zuvor genannte Druckausgleichsmittel, wie insbesondere die Entlastungsöffnung, ein Sperrelement aufweisen. Das Sperrelement kann beispielsweise als ein Filter ausgestaltet sein. Durch das Sperrelement kann das Austreten von Flüssigkeit aus dem Inneren des Gehäuses verhindert werden. Nach einer Kopplung der Fließtest-Einheit mit einem weiteren Behältnis kann somit ein geschlossenes System ausgebildet sein, in welchem vorzugsweise ein dem Umgebungsdruck identischer Druck herrscht. Wenigstens erfolgt jedoch ein Druckausgleich zwischen dem Innendruck des Behältnisses, insbesondere dem Innendruck eines Probenaufnahmeraums des Behältnisses, und dem Inneren, insbesondere den Kammern, des Gehäuses der Fließtest-Einheit.

Die Erfindung betrifft zudem ein Set aus einer Fließtest-Einheit, wie sie hierin beschrieben und beansprucht ist, und einem Probenbehälter, wobei der Probenbehälter eine zur Kupplungsstelle der Fließtest-Einheit passende Gegenkupplungsstelle aufweist. Es kann somit ein einheitlicher Probenbehälter mit einer Anzahl von unterschiedlichen Fließtest-Einheiten und/oder eine einheitliche Fließtest-Einheit mit unterschiedlichen Probenbehältern kombinierbar ausgebildet sein. Alternativ oder zusätzlich ist es möglich, eine Fließtest-Einheit zu unterschiedlichen Zeitpunkten am Probenbehälter anzubringen. Hierbei kann vorgesehen sein, dass der Probenbehälter eine Sollbruchstelle aufweist, die bei einer Kopplung der Fließtest-Einheit mit dem Probenbehälter durch ein Durchbruchelement der Fließtest-Einheit durchbrochen wird, so dass über die Zuführöffnung eine Flüssigkeitsverbindung zwischen dem Probenbehälter und der Fließtest-Einheit hergestellt wird. Wie bereits zuvor in Bezug auf die Fließtest-Einheit ausführlich beschrieben wurde, weist das Set den Vorteil auf, dass eine in einem abgeschlossenen Probenbehälter, insbesondere in einem hermetisch abgedichteten Probenbehälter, enthaltene Probe mittels der Fließtest-Einheit auf das Vorhandensein einer bestimmten Substanz untersucht werden kann, ohne dass die Probe aus dem abgeschlossenen System, insbesondere dem hermetisch abgeschlossenen System, entnommen oder eine Verbindung zwischen dem geschlossenes System mit der Umgebung hergestellt werden muss, beispielsweise indem das System kurzzeitig geöffnet werden muss. Durch die Kopplung der Fließtest-Einheit mit dem Probenbehälter strömt Flüssigkeit über die eingerichtete Flüssigkeitsverbindung in die Fließtest-Einheit und benetzt den Teststreifen. Somit kann eine Nachweisreaktion in Gang gesetzt werden, die innerhalb des abgedichteten Gehäuses, insbesondere innerhalb des hermetisch abgedichteten Gehäuses, der Fließtest-Einheit erfolgt.

Die Erfindung betrifft somit zudem die Verwendung einer Fließtest-Einheit, wie sie hierin beschrieben und beansprucht ist, zur Durchführung einer Nachweisreaktion bei einer Probe. Vorzugsweise handelt es sich dabei um eine Probe, die in einem mit der Fließtest-Einheit gekoppelten Probenbehälter bereitgehalten wird, wobei zwischen der Fließtest-Einheit und dem Probenbehälter eine Flüssigkeitsverbindung eingerichtet wird.

Die Erfindung betrifft also ferner die Verwendung einer Fließtest-Einheit, wie sie hierin beschrieben und beansprucht ist, zur Kopplung mit einem Probenbehälter, insbesondere wobei eine Verbindungsstelle zur Herstellung einer Flüssigkeitsverbindung zwischen der Fließtest-Einheit und dem Probenbehälter flüssigkeitsdicht ist.

Die Erfindung wird nun anhand mehrerer Ausführungsbeispiele näher beschrieben, ist jedoch nicht auf diese Ausführungsbeispiele beschränkt. Weitere Ausführungsbeispiele ergeben sich durch die Kombination der Merkmale einzelner oder mehrerer Ansprüche untereinander und/oder mit einzelnen oder mehreren Merkmalen der Ausführungsbeispiele.

Es zeigt:
- Fig. 1: eine mögliche Ausführungsvariante einer erfindungsgemäßen Fließtest-Einheit in längsgeschnittener Ansicht,
- Fig. 2: eine perspektivische Ansicht der Fließtest-Einheit aus Fig. 1,
- Fig. 3: eine mögliche Ausführungsvariante eines erfindungsgemäßen Sets aus einer Fließtest-Einheit und einem Probenbehälter in noch nicht gekoppelten Zustand,
- Fig. 4: den Ablauf einer Kopplung der Fließtest-Einheit und des Probenbehälters über die Zuführöffnung der Fließtest-Einheit und einer Sollbruchstelle in einer Außenwandung des Probenbehälters, wobei es nach dem Durchstoßen der Sollbruchstelle mithilfe der Zuführöffnung zu einem Einströmen von Flüssigkeit aus dem insbesondere unter Druck stehenden Probenbehälter in das Innere des Gehäuses der Fließtest-Einheit kommt,
- Fig. 5: eine Ausführungsvariante eines erfindungsgemäßen Sets aus mehreren Fließtest-Einheiten und einem Probenbehälter, wobei jede Fließtest-Einheit eine Kupplungsstelle aufweist, die dazu eingerichtet ist, mit einer zur insbesondere mechanischen Kupplungsstelle passenden, insbesondere mechanischen, Gegenkupplungsstelle an der Außenwandung des Probenbehälters verrastet zu werden, um die Fließtest-Einheiten fest mit dem Probenbehälter zu verbinden.
- Fig. 6: den Ablauf der Kopplung zwischen einer FließtestEinheit und einem Probenbehälter, wobei hier im Detail ein insbesondere irreversibles Verrasten der Fließtest-Einheit an der Außenwandung des Probenbehälters gezeigt ist.

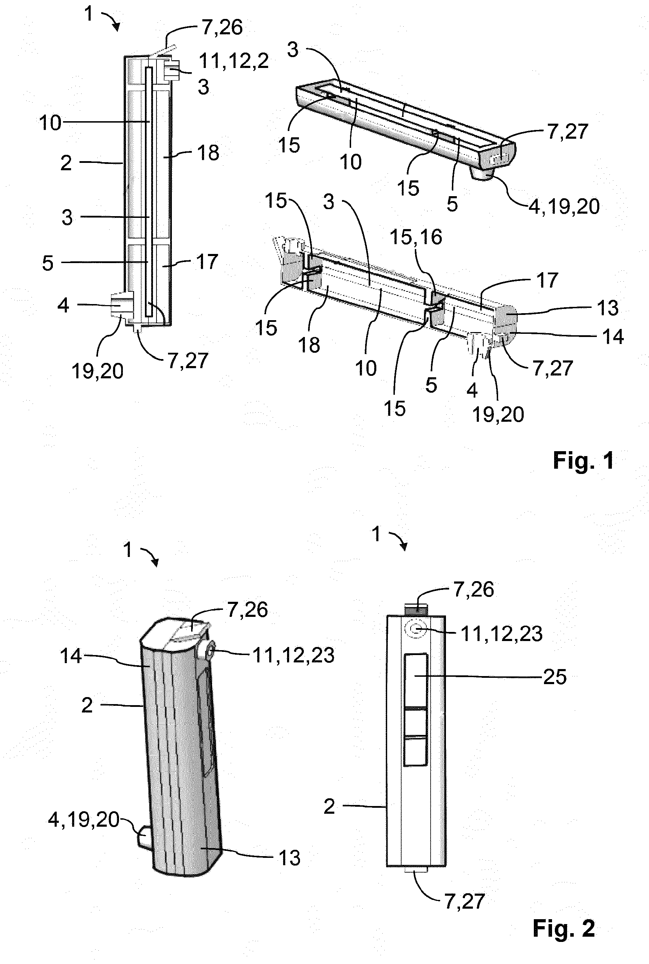

In den Figuren 1-6 ist eine im Ganzen jeweils als 1 bezeichnete Fließtest-Einheit gezeigt.

Die Fließtest-Einheit 1 ist dazu geeignet, um eine Analyse einer Probe in einem nach außen hin geschlossenen Gehäuse 2 der Fließtest-Einheit 1 vornehmen zu können.

In dem Gehäuse 2 ist ein Teststreifen 3 eingelegt, der durch das Gehäuse 2 umgeben ist. Insbesondere kann es sich dabei um ein insbesondere in Kopplungsstellung mit einem Probenbehälter 9 flüssigkeitsdicht abgeschlossenes oder abschließbares Gehäuse 2 handeln.

Der Teststreifen 3 ist zum Durchführen einer Nachweisreaktion vorgesehen. Dabei kann es sich beispielsweise um ein Teststreifen 3 handeln, der aus dem Stand der Technik bereits bekannt ist. Insbesondere kann es sich dabei um einen Teststreifen 3 handeln, der wenigstens aus einem Träger und wenigstens einer darauf angeordneten Fließ-Lage aufgebaut ist. In der Fließ-Lage kann eine zu Durchführung einer Nachweisreaktion erforderliche Substanz eingebracht sein, die als Reaktionspartner für einen nachzuweisenden Stoff in einer Probe und/oder als Wechselwirkungspartner für den Stoff dient. Durch eine in Kontaktbringung der zum Nachweis erforderlichen Substanz mit dem nachzuweisenden Stoff wird eine Nachweisreaktion in Gang gesetzt, deren Ergebnis durch einen Anwender vorzugweise direkt vom Teststreifen oder indirekt mittels einer Hilfsvorrichtung ablesbar ist.

Das Gehäuse 2 weist wenigstens eine Zuführöffnung 4 auf, die von außen in eine Einströmkammer 17 des Gehäuses 2 führt. Dabei kann es vorgesehen sein, dass die Zuführöffnung 4 in nicht gekoppeltem Zustand der Fließtest-Einheit 1 verschlossen ist. Beispielsweise kann die Zuführöffnung 4 durch eine dünne Wandung, welche als eine Sollbruchstelle 21 eingefügt ist, verschlossen sein. Die Sollbruchstelle 21 kann hierbei beispielsweise durch einen Kopplungsvorgang mit einem Probenbehälter 9 durchstoßen werden, so dass eine Flüssigkeitsverbindung 22 zwischen der Fließtest-Einheit 1 und einem Probenaufnahmeraum des Probenbehälters 9 ausgebildet wird.

Ein Benetzungsbereich 5 des Teststreifens 3 ist daher derart innerhalb des Gehäuses 2 angeordnet, dass der Benetzungsbereich 5 mit in das Innere des Gehäuses 2 durch die Zuführöffnung 4 einströmender Flüssigkeit in Kontakt kommt. Dadurch kann eine Nachweisreaktion in Gang gesetzt werden.

An der Außenseite des Gehäuses 2 ist eine Kupplungsstelle 7 ausgebildet, über welche die Fließtest-Einheit 1 mit einer dazu passenden Gegenkupplungsstelle 8 einer Wandung des Probenbehälters 9 koppelbar ist, insbesondere also mechanisch verbindbar ist. Somit ist es möglich, die Fließtest-Einheit 1 mit dem Probenbehälter 9 irreversibel zu koppeln. Irreversibel kann in diesem Zusammenhang bedeuten, dass bei einer sachgemäßen Benutzung keine Trennung des Probenbehälters 9 von der Fließtest-Einheit 1 möglich ist. Insbesondere kann dabei vorgesehen sein, dass kein Auslösemechanismus an der Kupplungsstelle 7 und/oder der Gegenkupplungsstelle 9 ausgebildet ist, um deren Kopplung aufzuheben.

Der Teststreifen 3 kann neben dem zuvor bereits genannten Benetzungsbereich 5 einen weiten Bereich aufweisen, der als Analysebereich 10 bezeichnet werden kann. In diesem Bereich, der vorzugsweise während des Gebrauchs nicht direkt mit Flüssigkeit in Kontakt tritt, findet die Nachweisreaktion statt. Dabei kann die Flüssigkeit 6 durch den Benetzungsbereich 5 vorzugsweise durch Kapillarkräfte angesaugt und bis in den Analysebereich 10 transportiert werden. Zum Beispiel kann der Teststreifen zur Durchführung eines lateralen Fließtests (engl. Lateral Flow Test) vorgesehen sein, wie dieser insbesondere bereits aus dem Stand der Technik bekannt ist.

Wie in den Figuren 1 und 2 gezeigt ist, weist die Fließtest-Einheit 1 am Gehäuse 2 ein Druckausgleichsmittel 11 auf. Das Druckausgleichsmittel 11 ist dazu eingerichtet, um einen beispielsweise in einem Probenbehälter 9 herrschenden Überdruck ausgleichen zu können, insbesondere bis sich im Probenbehälter 9 ein atmosphärischer Außendruck und/oder ein Druckausgleich zwischen dem Druck im Probenbehälter 9 und dem Druck in der Fließtest-Einheit 1 eingestellt hat, nachdem der Probenbehälter 9 mit der Fließtest-Einheit 1 gekoppelt wurde.

Wie in den Figuren 1 und 2 zu erkennen ist, kann das Druckausgleichsmittel 11 beispielsweise als eine Entlastungsöffnung 12 ausgebildet sein, über die ein Überdruck nach außen entweichen kann. Das Druckausgleichsmittel 11 kann an einem gegenüberliegenden Ende zu dem Ende ausgebildet sein, an welchem die Zuführöffnung 4 ausgebildet ist. Daher können Druckausgleichsmittel 11 und Zuführöffnung 4 in axialer Richtung beabstandet zueinander angeordnet sein. Insbesondere kann das Druckausgleichsmittel 11 eine Wandung der Analysekammer 18 durchbrechen und/oder an einer Wandung der Analysekammer 18 ausgebildet sein.

Wie in Figur 2 gezeigt ist, weist das Gehäuse 2 ein erstes Gehäuseteil 13 und ein zweites Gehäuseteil 14 auf, die in zusammengesetztem Zustand das nach außen hin geschlossene, wenigstens in Kopplungsstellung flüssigkeitsdichte Gehäuse 2 ausbilden.

Der Teststreifen 3 ist zwischen den beiden Gehäuseteilen 13, 14 eingelegt und wird dabei durch mehrere Haltestege 15 korrekt positioniert.

Dabei ist der Teststreifen 3 zwischen zwei Paaren aus jeweils zwei Haltestegen 15 eingesetzt. Der Abstand zwischen einem Paar von zwei Haltestegen 15 ist dabei größer gewählt, als eine Wandstärke und/oder Dicke des Teststreifens 3. Somit kann gewährleistet werden, dass der Teststreifen 3 durch die Haltestege 15 nicht gequetscht ist, so dass Flüssigkeit 6 ungehindert an den Haltestegen 15 durch den Teststreifen 3 vorbeiströmen kann. Der Teststreifen 3 wird somit durch die Haltestege 15 gehalten und seitlich fixiert.

Das Innere des Gehäuses 2 ist, wie in den Figuren 1,4 und 6 gezeigt ist, durch eine als Prallschutz 16 ausgebildete Wandung in zwei Kammern 17, 18 unterteilt. Eine Einströmkammer 17 weist die zuvor genannte Zuführöffnung 4 auf, wobei der Benetzungsbereich 5 innerhalb der Einströmkammer 17 angeordnet ist. Die Analysekammer 18 weist die Entlastungsöffnung 12 auf, wobei der Analysebereich 10 des Teststreifens 3 in der Analysekammer 18 angeordnet ist.

Durch die Ausbildung des Prallschutzes 16 ist es möglich, zu verhindern, dass es durch ein schwallartiges Einströmen von Flüssigkeit 6 aus einem beispielsweise unter Druck stehenden Probenbehälter 9 in die Fließtest-Einheit 1 zu einer ungewollten Benetzung des Analysebereichs 10 des Teststreifens 3 kommt. Einströmende Flüssigkeit prallt daher am Prallschutz 16 ab zurück in die Einströmkammer 17. Nach Einstellung eines Druckausgleiches ist es vorgesehen, dass der Flüssigkeitspegel der Flüssigkeit 6 innerhalb des Gehäuses 2 unterhalb des Prallschutzes 16 liegt, also nicht bis in die Analysekammer 18 ansteigt.

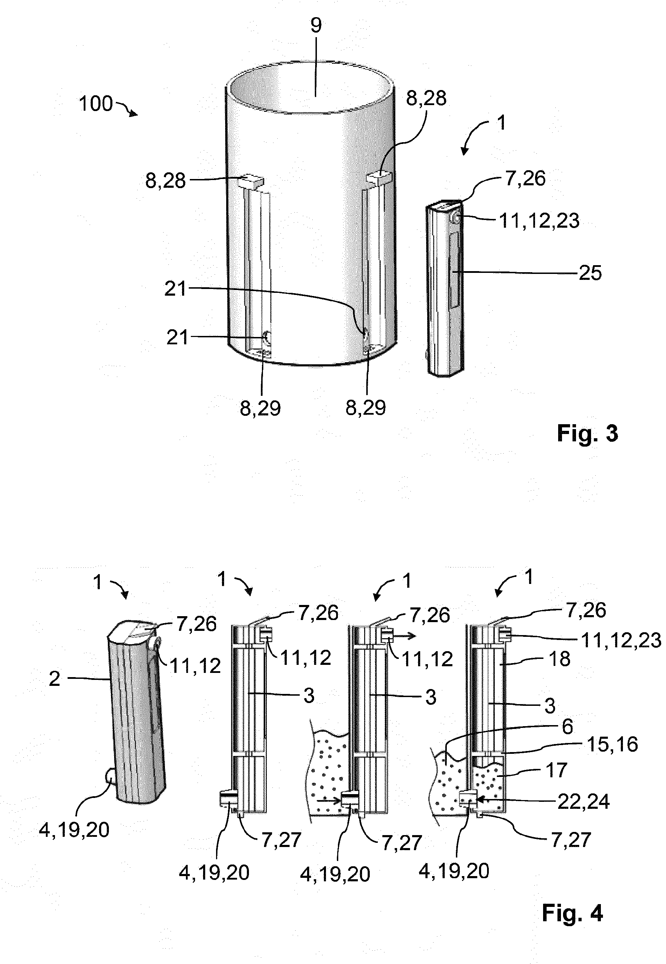

Das Gehäuse 2 der Fließtest-Einheit 1 ist wenigstens teilweise transparent ausgestaltet, so dass ein Anwender Sicht auf den Teststreifen 3 hat, insbesondere auf den Analysebereich 10 des Teststreifens 3, um ein Ergebnis der Nachweisreaktion ablesen zu können. Figur 3 zeigt eine Ausführungsvariante, die ein Sichtfenster aufweist, das eine Sicht von außerhalb des Gehäuses 2 auf den Teststreifen 3 bietet.

Die Zuführöffnung 4 ist als ein Durchbruchelement 19 ausgebildet, das schräg oder senkrecht bezogen auf eine Längsachse der Fließtest-Einheit 1 vor einer Außenwandung des Gehäuses 2 absteht.

Der Probenbehälter 9 kann an einer Außenwandung eine Sollbruchstelle 21 aufweisen.

Bei einer Kopplung der Fließtest-Einheit 1 mit dem Probenbehälter 9 beaufschlagt das Durchbruchelement 19 die Sollbruchstelle 21 und durchbricht diese. Dabei wird eine Flüssigkeitsbindung 22 zwischen einem Probenaufnahmeraum des Probenbehälters 9 und der Einströmkammer 17 der Fließtest-Einheit 1 hergestellt. Die Verbindungsstelle zwischen Fließtest-Einheit 1 und dem Probenbehälter 9 ist durch die anliegende Reibungskraft zwischen den sich kontaktierenden Teilen ausreichend abgedichtet, um einem Druck standzuhalten, der beispielsweise über dem atmosphärischen Druck liegt und innerhalb des Probenbehälters 9 herrscht. Alternativ oder ergänzend dazu kann an der Verbindungsstelle wenigstens ein zusätzliches Dichtmittel angeordnet sein.

Das Durchbruchelement 19 kann beispielsweise als eine in radialer und/oder axialer Richtung abstehende Nase 20 ausgebildet sein.

Wie bereits zuvor erläutert, kann auch die Zuführöffnung 4 eine Sollbuchstelle 21 aufweisen, so dass das Gehäuse 2 in nicht gekoppeltem Zustand nach außen geschlossen ist, insbesondere hermetisch geschlossen ist. Durch den Kopplungsvorgang kann somit auch diese Sollbruchstelle durch ein weiteres Durchbruchelement 19, das zum Beispiel an der Außenwandung des Probenbehälters 9 ausgestaltet ist, durchbrochen werden. Diese Ausführungsvariante ist in den Figuren jedoch nicht gezeigt.

Um jegliche Kontamination der Umgebung durch eine zu analysierende Probe, die in einer Flüssigkeit 6 enthalten ist, ausschließen zu können, kann das Druckausgleichsmittel 11 ein Sperrelement 23 aufweisen, durch welches wenigstens Flüssigkeit 6 zurückgehalten wird. Beispielsweise kann es sich dabei um einen Filter handeln, dessen Maschenbreite entsprechend gewählt ist, um flüssigkeitsundurchlässig zu sein, sodass lediglich Gase entweichen können.

Bei den in den Figuren 3, 4, 5 und 6 gezeigtem Set 100 aus Fließtest-Einheit 1 und Probenbehälter 9 ist die Kupplungsstelle 7 der Fließtest-Einheit 1 korrespondierend zur Gegenkupplungsstelle 8 an der Außenwandung des Probenbehälters 9 ausgebildet.

Die Kupplungsstelle 7 weist ein an einem Ende des Gehäuses 2 schräg abstehendes, vorgespanntes Federelement 26 auf. Das Federelement 26 ist dazu eingerichtet, eine Federkraft zu entwickeln, die überwunden werden muss, um die Fließtest-Einheit 1 an einen Probenbehälter 9 anzukoppeln. Die Kupplung 7 weist an einem gegenüberliegenden Ende des Gehäuses 2 ein Rastelement 27 auf. Z.B. kann das Rastelement 27 als eine axial abstehende Rastnase ausgebildet sein.

Die Gegenkupplungsstelle 8 weist ein zum Federelement 26 korrespondierendes Gegenrastelement 28 auf. Beispielsweise kann das Gegenrastelement 28 als ein insbesondere steifer Rasthaken ausgebildet sein, der im gekoppelten Zustand das Federelement 26 wenigstens teilweise überragt und/oder beaufschlagt und/oder zusammendrückt.

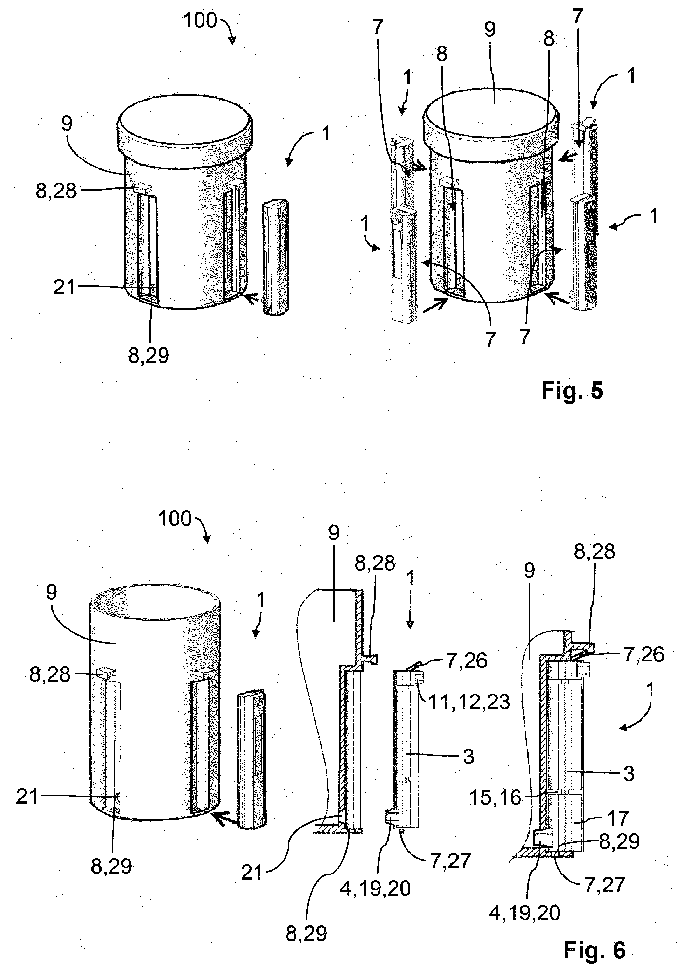

Des Weiteren weist die Gegenkupplungsstelle 8 eine Ausnehmung 29 auf, die beispielsweise in einer radialen Verlängerung des Bodens des Probenbehälters 9 ausgebildet sein kann, wie in den Figuren 4 und 6 dargestellt ist.

Im gekoppelten Zustand greift das Rastelement 27 in die Ausnehmung 29 ein. Vorzugsweise kann dadurch eine Drehachse ausgebildet werden, wobei die Fließtest-Einheit 1 wie ein Hebel um die Drehachse in Richtung der Außenwandung des Probenbehälters 9 bewegbar ist, bis eine Flüssigkeitverbindung 22 und eine Verrastung zwischen dem Federelement 26 und dem Gegenrastelement 28 hergestellt ist. Der dafür erforderliche Kraftaufwand, um die wenigstens eine Sollbruchstelle 21 zu durchbrechen, ist aufgrund der Hebelwirkung relativ gering.

Die Erfindung betrifft also insbesondere eine Fließtest-Einheit 1, mit einem nach außen zumindest in einer Kopplungsstellung mit einem Probenbehälter 9 dicht verschlossenen Gehäuse 2, wobei das Gehäuse 2 wenigstens einen Teststreifen 3 umfasst, wobei das Gehäuse 2 wenigstens eine Zuführöffnung 4 aufweist, und wobei der Teststreifen 3 innerhalb des Gehäuses 2 so angeordnet ist, dass nach der Herstellung einer Flüssigkeitsverbindung 22 mit einem Probenbehälter 9 ein Benetzungsbereich 5 mit über die wenigstens eine Zuführöffnung 4 eindringender Flüssigkeit 6 benetzbar ist.

### Bezugszeichenliste

- 1: Fließtest-Einheit
- 2: Gehäuse
- 3: Teststreifen
- 4: Zuführöffnung
- 5: Benetzungsbereich
- 6: Flüssigkeit (inkl. des zu analysierenden Probenmaterials)
- 7: Kupplungsstelle
- 8: Gegenkupplungsstelle
- 9: Probenbehälter
- 10: Analysebereich
- 11: Druckausgleichsmittel
- 12: Entlastungsöffnung
- 13: Erstes Gehäuseteil
- 14: Zweites Gehäuseteil
- 15: Haltestege
- 16: Prallschutz
- 17: Einströmkammer
- 18: Analysekammer
- 19: Durchbruchelement
- 20: Nase
- 21: Sollbruchstelle
- 22: Flüssigkeitsverbindung
- 23: Sperrelement
- 24: Verbindungsstelle
- 25: Sichtfenster
- 26: Federelement
- 27: Rastelement
- 28: Gegenrastelement
- 29: Ausnehmung
- 100: Set

## Patentansprüche

1. Fließtest-Einheit (1), mit einem nach außen geschlossenen Gehäuse (2), welches wenigstens einen Teststreifen (3) umfasst, wobei das Gehäuse (2) wenigstens eine Zuführöffnung (4) aufweist, und wobei der Teststreifen (3) innerhalb des Gehäuses (2) so angeordnet ist, dass ein Benetzungsbereich (5) mit über die wenigstens eine Zuführöffnung (4) eindringender Flüssigkeit (6) benetzbar ist, und das Gehäuse (2) an einer Außenseite eine Kupplungsstelle (7) aufweist, über welche die Fließtest-Einheit (1) mit einer dazu passenden Gegenkupplungsstelle (8) einer Wandung eines Probenbehälters (9) koppelbar ist, **dadurch gekennzeichnet, dass** das Gehäuse (2) ein Druckausgleichsmittel (11) aufweist, um ein Einströmen von Flüssigkeit (6) zu befördern, wobei das Druckausgleichsmittel (11) als ein Auffangsack ausgestaltet ist und/oder wobei das Gehäuse (2) mit einem Unterdruck versehen ist.

2. Fließtest-Einheit (1) nach Anspruch 1, 1 , **dadurch gekennzeichnet, dass** der wenigstens eine Teststreifen (3) in einen Benetzungsbereich (5) und einen Analysebereich (10) unterteilt ist, insbesondere wobei eine Nachweisreaktion in Gang setzbar ist, wenn der Benetzungsbereich (5) mit einer zu analysierenden Flüssigkeit (6) in Kontakt tritt und/oder dass der Teststreifen (3) zur Durchführung eines lateralen Fließtests (Lateral Flow Test) ausgestaltet ist.

3. Fließtest-Einheit (1) nach einem der vorstehenden Ansprüche, **dadurch gekennzeichnet, dass** das Gehäuse (2) zumindest zweiteilig oder genau zweiteilig ausgebildet ist, insbesondere wobei der Teststreifen (3) zwischen den wenigstens zwei Gehäuseteilen (13, 14) eingelegt ist.

4. Fließtest-Einheit (1) nach einem der vorstehenden Ansprüche, **dadurch gekennzeichnet, dass** der wenigstens eine Teststreifen (3) durch wenigstens einen Haltesteg (15) des Gehäuses (2) gehalten ist und/oder seitlich fixiert ist, vorzugsweise derart, dass der wenigstens eine Teststreifen (3) anliegt und/oder nicht durch den wenigstens einen Haltesteg (15) deformiert ist.

5. Fließtest-Einheit (1) nach einem der vorstehenden Ansprüche, **dadurch gekennzeichnet, dass** das Gehäuse (2) im Inneren einen Prallschutz (16) aufweist, durch welchen das Gehäuse (2) in zwei Kammern (17, 18) unterteilt ist, insbesondere wobei der Benetzungsbereich (5) des wenigstens einen Teststreifens (3) in einer Einströmkammer (17) angeordnet ist und der Analysebereich (18) des wenigstens einen Teststreifens (3) in einer Analysekammer (18) des Gehäuses (2) angeordnet ist.

6. Fließtest-Einheit (1) nach einem der vorstehenden Ansprüche, **dadurch gekennzeichnet, dass** das Gehäuse (2) wenigstens teilweise transparent ausgebildet ist und/oder dass das Gehäuse (2) eine Markierung aufweist, über welche durch einen Anwender ein Ergebnis einer Analyse ablesbar ist.

7. Fließtest-Einheit (1) nach einem der vorstehenden Ansprüche, **dadurch gekennzeichnet, dass** die wenigstens eine Zuführöffnung (4) als ein Durchbruchelement (19), insbesondere als eine radial und/oder axial abragende Nase (20), ausgebildet ist, mittels welcher eine Sollbruchstelle (21) in einer Wandung durch einen Kopplungsvorgang der Fließtest-Einheit (1) mit der Wandung durchbrechbar ist, insbesondere so dass über die Zuführöffnung (4) eine Flüssigkeitsverbindung (22) mit dem Probenbehälter (9) hergestellt ist.

8. Fließtest-Einheit (1) nach einem der vorstehenden Ansprüche, **dadurch gekennzeichnet, dass** ein oder das Druckausgleichsmittel (11) ein Sperrelement (23) aufweist, vorzugsweise einen Filter aufweist, um das Austreten von Flüssigkeit (6) aus dem Inneren des Gehäuses (2) zu verhindern.

9. Set (100) aus einer Fließtest-Einheit (1) nach einem der vorstehenden Ansprüche und einem Probenbehälter (9), **dadurch gekennzeichnet, dass** der Probenbehälter (9) eine zur Kupplungsstelle (7) der Fließtest-Einheit (1) passende Gegenkupplungsstelle (8) aufweist, insbesondere wobei der Probenbehälter (9) eine Sollbruchstelle (21) aufweist, die bei einer Kopplung der Fließtest-Einheit (1) mit dem Probenbehälter (9) durch ein Durchbruchelement (19) der Fließtest-Einheit (1) durchbrochen wird, so dass über die Zuführöffnung (4) eine Flüssigkeitsverbindung (22) zwischen dem Probenbehälter (9) und der Fließtest-Einheit (1) hergestellt wird.

10. Verwendung einer Fließtest-Einheit (1) nach einem der vorstehenden Ansprüche 1 bis 8, zur Durchführung einer Nachweisreaktion bei einer Probe, vorzugsweise wobei die Probe in einem mit der Fließtest-Einheit (1) gekoppelten Probenbehälter (9) bereitgehalten wird, wobei zwischen der Fließtest-Einheit (1) und dem Probenbehälter (9) eine Flüssigkeitsverbindung (22) hergestellt ist.

11. Verwendung einer Fließtest-Einheit (1) nach einem der vorstehenden Ansprüche, zur Kopplung mit einem Probenbehälter (9), insbesondere wobei eine Verbindungsstelle (24) zur Herstellung einer Flüssigkeitsverbindung (22) zwischen der Fließtest-Einheit (1) und dem Probenbehälter (9) flüssigkeitsdicht ist.

## Claims

1. Flow test unit (1) with a housing (2) which is closed to the outside and which comprises at least one test strip (3), wherein the housing (2) has at least one admission opening (4), and wherein the test strip (3) is arranged inside the housing (2) such that a wetting region (5) can be wetted with liquid (6) entering via the at least one admission opening (4), and the housing (2) has, on an outer face, a coupling point (7) via which the flow test unit (1) can be coupled to a matching counter-coupling point (8) of a wall of a sample container (9), **characterized in that** the housing (2) has a pressure-equalizing means (11) in order to promote an inward flow of liquid (6), wherein the pressure-equalizing means (11) is designed as a collecting pouch, and/or wherein the housing (2) is provided with an underpressure.

2. Flow test unit (1) according to Claim 1, **characterized in that** the at least one test strip (3) is divided into a wetting region (5) and an analysis region (10), in particular wherein a detection reaction can be set in motion when the wetting region (5) comes into contact with a liquid (6) to be analysed, and/or **in that** the test strip (3) is designed for performing a lateral flow test.

3. Flow test unit (1) according to one of the preceding claims, **characterized in that** the housing (2) is configured in at least two parts or precisely in two parts, in particular wherein the test strip (3) is placed between the at least two housing parts (13, 14).

4. Flow test unit (1) according to one of the preceding claims, **characterized in that** the at least one test strip (3) is held and/or laterally fixed by at least one holding web (15) of the housing (2), preferably in such a way that the at least one test strip (3) rests thereon and/or is not deformed by the at least one holding web (15).

5. Flow test unit (1) according to one of the preceding claims, **characterized in that** the housing (2) has, in its interior, a baffle (16) by which the housing (2) is divided into two chambers (17, 18), in particular wherein the wetting region (5) of the at least one test strip (3) is arranged in an inflow chamber (17) and the analysis region (18) of the at least one test strip (3) is arranged in an analysis chamber (18) of the housing (2).

6. Flow test unit (1) according to one of the preceding claims, **characterized in that** the housing (2) is at least partially transparent, and/or **in that** the housing (2) has a marking allowing a user to read off a result of an analysis.

7. Flow test unit (1) according to one of the preceding claims, **characterized in that** the at least one admission opening (4) is configured as a piercing element (19), in particular as a radially and/or axially protruding lug (20), by means of which a predetermined breaking point (21) in a wall can be pierced through in a process of coupling the flow test unit (1) to the wall, in particular such that a liquid connection (22) to the sample container (9) is produced via the admission opening (4).

8. Flow test unit (1) according to one of the preceding claims, **characterized in that** a or the pressure-equalizing means (11) has a blocking element (23), preferably a filter, in order to prevent liquid (6) from leaving the interior of the housing (2).

9. Kit (100) composed of a flow test unit (1), according to one of the preceding claims, and a sample container (9), **characterized in that** the sample container (9) has a counter-coupling point (8) matching the coupling point (7) of the flow test unit (1), in particular wherein the sample container (9) has a predetermined breaking point (21) which, when the flow test unit (1) is coupled to the sample container (9), is pierced through by a piercing element (19) of the flow test unit (1), such that a liquid connection (22) between the sample container (9) and the flow test unit (1) is produced via the admission opening (4).

10. Use of a flow test unit (1) according to one of Claims 1 to 8, for performing a detection reaction on a sample, preferably wherein the sample is made available in a sample container (9) coupled to the flow test unit (1), wherein a liquid connection (22) is produced between the flow test unit (1) and the sample container (9).

11. Use of a flow test unit (1) according to one of the preceding claims, for coupling to a sample container (9), in particular wherein a connecting point (24) for producing a liquid connection (22) between the flow test unit (1) and the sample container (9) is impermeable to liquid.

## Revendications

1. Unité d'essai d'écoulement (1) dotée d'un boîtier (2) fermé vers l'extérieur qui comprend au moins une bande de test (3), dans laquelle le boîtier présente au moins une ouverture d'alimentation (4) et dans laquelle la bande de test (3) est disposée à l'intérieur du boîtier (2) de telle sorte qu'une zone de mouillage (5) peut être mouillée avec un liquide (6) pénétrant par l'au moins une ouverture d'alimentation (4), et le boîtier (2) présente sur une face extérieure un point d'accouplement (7) par lequel l'unité d'essai d'écoulement (1) peut être accouplée avec un point d'accouplement homologue correspondant (8) d'une paroi d'un récipient d'échantillon (9), **caractérisée en ce que** le boîtier (2) comporte un moyen de compensation de pression (11) pour favoriser un afflux de liquide (6), le moyen de compensation de pression (11) étant configuré comme un sac collecteur et/ou le boîtier (2) étant en dépression.

2. Unité d'essai d'écoulement (1) selon la revendication 1, **caractérisée en ce que** l'au moins une bande de test (3) est divisée en une zone de mouillage (5) et une zone d'analyse (10), une réaction de détection pouvant en particulier être mise en route quand la zone de mouillage (5) entre en contact avec un liquide (6) à analyser et/ou que la bande de test (3) est conçue pour réaliser un essai d'écoulement latéral (latéral flow test).

3. Unité d'essai d'écoulement (1) selon une des revendications précédentes, **caractérisée en ce que** le boîtier (2) est configuré en au moins deux parties ou exactement en deux parties, en particulier la bande de test (3) étant insérée entre les au moins deux parties de boîtier (13, 14).

4. Unité d'essai d'écoulement (1) selon une des revendications précédentes, **caractérisée en ce que** l'au moins une bande de test (3) est maintenue par au moins un tenon de maintien (15) du boîtier (2) et/ou est fixée latéralement, de préférence de telle sorte que l'au moins une bande de test (3) est positionnée et/ou n'est pas déformée par l'au moins un tenon de maintien (15).

5. Unité d'essai d'écoulement (1) selon une des revendications précédentes, **caractérisée en ce que** le boîtier (2) comporte à l'intérieur une protection antichoc (16) par laquelle le boîtier (2) est divisé en deux chambres (17, 18), en particulier la zone de mouillage (5) de l'au moins une bande de test (3) étant disposée dans une chambre d'admission (17) et la zone d'analyse (10) de l'au moins une bande de test (3) étant disposée dans une chambre d'analyse (18) du boîtier.

6. Unité d'essai d'écoulement (1) selon une des revendications précédentes, **caractérisée en ce que** le boîtier (2) est au moins partiellement transparent et/ou que le boîtier (2) présente un marquage par le biais duquel un résultat d'une analyse peut être lu par un utilisateur.

7. Unité d'essai d'écoulement (1) selon une des revendications précédentes, **caractérisée en ce que** l'au moins une ouverture d'alimentation (4) est configurée comme un élément de rupture (19), en particulier comme un bec (20) saillant radialement et/ou axialement au moyen duquel un point de rupture imposé (21) dans une paroi peut être brisé par un processus d'accouplement de l'unité d'essai d'écoulement (1) avec la paroi, en particulier de telle sorte qu'une communication de liquide (22) est établie avec le récipient d'échantillon (9) via l'ouverture d'alimentation (4).

8. Unité d'essai d'écoulement (1) selon une des revendications précédentes, **caractérisée en ce qu'**un ou le moyen de compensation de pression (11) comporte un élément de blocage (23), de préférence comporte un filtre, pour empêcher le liquide (6) de sortir de l'intérieur du boîtier (2).

9. Kit (100) constitué d'une unité d'essai d'écoulement (1) selon une des revendications précédentes et d'un récipient d'échantillon (9), **caractérisé en ce que** le récipient d'échantillon comporte un point d'accouplement homologue (8) adapté au point d'accouplement (7) de l'unité d'écoulement d'essai (7), en particulier le récipient d'échantillon (9) présentant un point de rupture imposé (21) qui est brisé lors d'un accouplement de l'unité d'essai d'accouplement (1) avec le récipient d'échantillon (9) par un élément de rupture (19) de l'unité d'essai d'écoulement (1), de telle sorte qu'une communication de liquide (22) est établie entre le récipient d'échantillon (9) et l'unité d'écoulement d'essai (1) via l'ouverture d'alimentation 4.

10. Utilisation d'une unité d'essai d'écoulement (1) selon une des revendications précédentes 1 à 8 pour accomplir une réaction de détection sur un échantillon, l'échantillon étant de préférence conservé dans un récipient d'échantillon (9) accouplé à l'unité d'essai d'accouplement (1), une communication de liquide (22) étant établie entre l'unité d'écoulement d'essai (1) et le récipient d'échantillon (9).

11. Utilisation d'une unité d'essai d'écoulement (1) selon une des revendications précédentes pour l'accoupler avec un récipient d'échantillon (9), en particulier selon laquelle un point de communication (24) pour établir une communication de liquide (22) entre l'unité d'écoulement d'essai (1) et le récipient d'échantillon (9) est étanche aux liquides.
